Europäisches Patentamt

(19) European Patent Office

Office européen des brevets

(11) Publication number: 0 145 208
A1

# (12) EUROPEAN PATENT APPLICATION

(21) Application number: 84307381.8

(22) Date of filing: 26.10.84

(51) Int. Cl.⁴: A 61 B 5/02
A 61 B 7/04

(30) Priority: 04.11.83 JP 207077/83
04.11.83 JP 207078/83
04.11.83 JP 207083/83
04.11.83 JP 207084/83
09.11.83 JP 210306/83
10.11.83 JP 211654/83
20.04.84 JP 80973/84

(43) Date of publication of application:
19.06.85 Bulletin 85/25

(84) Designated Contracting States:
DE FR GB

(71) Applicant: SEIKO INSTRUMENTS & ELECTRONICS LTD.
31-1, Kameido 6-chome
Koto-ku Tokyo(JP)

(72) Inventor: Fukushima, Toshitaka
c/o Seiko Instruments & Electronics Ltd
6-31-1, Kameido Koto-ku Tokyo(JP)

(74) Representative: Caro, William Egerton et al,
J. MILLER & CO. Lincoln House 296-302 High Holborn
London WC1V 7JH(GB)

(54) An electronic sphygmomanometer.

(57) An electronic sphygmonanometer comprises a K-sound detection circuit (SD) for detecting Koroktoff sound from a microphone, a pressure transducer (17) for converting pressure to an electric signal, a pressure pulse detection circuit (20) for detecting a pressure pulse signal from the electric signal and a double integral type converter for converting an analog output of an amplifier (18) to a digital signal. An arithmetic unit (11) receives the output signals of the K-sound detection circuit (SD), the pressure pulse detection circuit (20) and the converter (8) drives a display device (12) for indicating systolic and diastolic pressures digitally. A detection circuit (S) includes the K-sound detection circuit (SD), the pressure transducer (17), the amplifier (18), the pressure pulse detection circuit (20) and an analog section (19a) of the converter. A logic circuit (L) includes a digital section (19b) of the converter (8), the arithmetic unit (11) and the digital display (12). A plurality of signal lines (WR) connected between the detection circuit (S) and the logic circuit (L) and include a first signal line (S5) for transmitting the output signal from the K-sound detection circuit to the arithmetic unit, a second signal line (S4) for transmitting the output of the pressure pulse detection circuit (20) to the analog section (19a) and a third signal line (S1, S3) for transmitting an output of the analog section (19a) to the digital section (19b).

./...

Croydon Printing Company Ltd.

EP 0 145 208 A1

# FIG.3

## AN ELECTRONIC SPHYGMOMANOMETER

This invention relates to electronic sphygmonanometers.

Electronic sphygmonanometers include an analog circuit and a logic circuit. The analog circuit includes transistors, all of which operate in the active condition and have a relatively high power consumption. A semiconductor pressure transducer is required to allow a relatively large current flow because its sensitivity is proportional to the value of the applied current. An operational amplifier is used in a pressure detection circuit and the elements of the operational amplifier are selected according to their offset voltage and temperature drift. Furthermore, noise has to be taken into account in the case of amplification of alternating current.

It is advantageous that the logic circuit of an electronic sphygmonanometer is actuated in the saturation position. Accordingly, the logic circuit is capable of being formed as a C-MOS circuit on a single chip.

Turning back to the analog circuit it has a large number of resistors and capacitors as additional circuit components as well as bipolar transistors.

It is difficult for conventional sphygmonanometers to be driven with a relatively low voltage and current because the amplitude of the signal voltage is not obtained in the low voltage and current.

The present invention seeks to provide a small size electronic sphygmonanometer using C-MOS logic IC and bipolar analog IC to operate at relatively low voltage with relatively small current consumption.

According to the present invention there is provided an electronic sphygmonanometer comprising: a K-sound detection circuit (SD) for detecting Korotkoff sound from a microphone; a pressure transducer for converting pressure to an electric signal; a pressure pulse detection circuit for detecting a pressure pulse signal from said electric signal, the detection circuit being connected to an amplifier through said pressure transducer; a double integral type convertor for converting an analog output of the amplifier to a digital signal; an arithmetic unit for receiving the output signals of said K-sound detection circuit said pressure pulse detection circuit and said convertor; and a display device for indicating systolic pressure and diastolic pressure digitally, characterised by detection means including said K-sound detection circuit, said pressure transducer, said amplifier, said pressure pulse detection circuit and an analog section of said convertor, a logic means including a digital section of said convertor, said arithmetic unit and said digital display, and a plurality of signal lines connected between said detection means and said logic means and including a first signal line for transmitting an output signal of said K-sound detection circuit to said arithmetic unit, a second signal line for transmitting said output of said pressure pulse detection circuit to said arithmetic section and a third signal line for transmitting an output of said analog section to said digital section.

Preferably said detection means and said logic means are formed on different IC chips.

Said signal line (S5) may be a bidirectional signal line.

In the preferred embodiment said analog section includes an electronic switch for switching between charging current and discharging current, said electronic switch comprising a voltage follower having a transistor, and an integrator having an integral capacitor, a first resistor and a second resistor connected in series, said integrator having one input terminal connected to the series arrangement of said first and second resistors and the other input terminal connected to a reference voltage source.

Said K-sound detection circuit may include a filter means for eliminating noise, a level detection circuit for determining level of detection of Korotkoff sound and connected to said filter and a feed back means connected between an output of said level detection circuit and said filter means.

In operation of the preferred embodiment discharge current value used for said convertor is proportional to the value of the constant current used for said pressure transducer.

The invention is illustrated, merely by way of example, in the accompanying drawings, in which:-

Figure 1 is a block diagram of a conventional electronic sphygmonanometer

Figure 2 is a graph illustrating the principle of measurement of blood pressure;

Figure 3 is a block diagram of one embodiment of an electronic sphygmonanometer according to the present invention;

Figures 4A and 4B are circuit diagrams of a detection circuit of the electronic sphygmonanometer of Figure 3;

Figure 5 is a circuit diagram of an operational amplifier of an analog section of an analog-to-digital converter of the electronic sphygmonanometer of Figure 3;

Figures 6 and 7 are timing charts showing outputs of the analog-to-digital convertor of Figure 5;

Figure 8 illustrates the operation of the detection circuit when a feedback circuit shown in Figure 4B is eliminated;

Figure 9 illustrates the operation of the detection circuit of Figure 4B; and

Figure 10 is a circuit diagram of a modification of the electronic sphygmonanometer of Figure 3.

Throughout the drawings like parts have been designated by the same reference numerals.

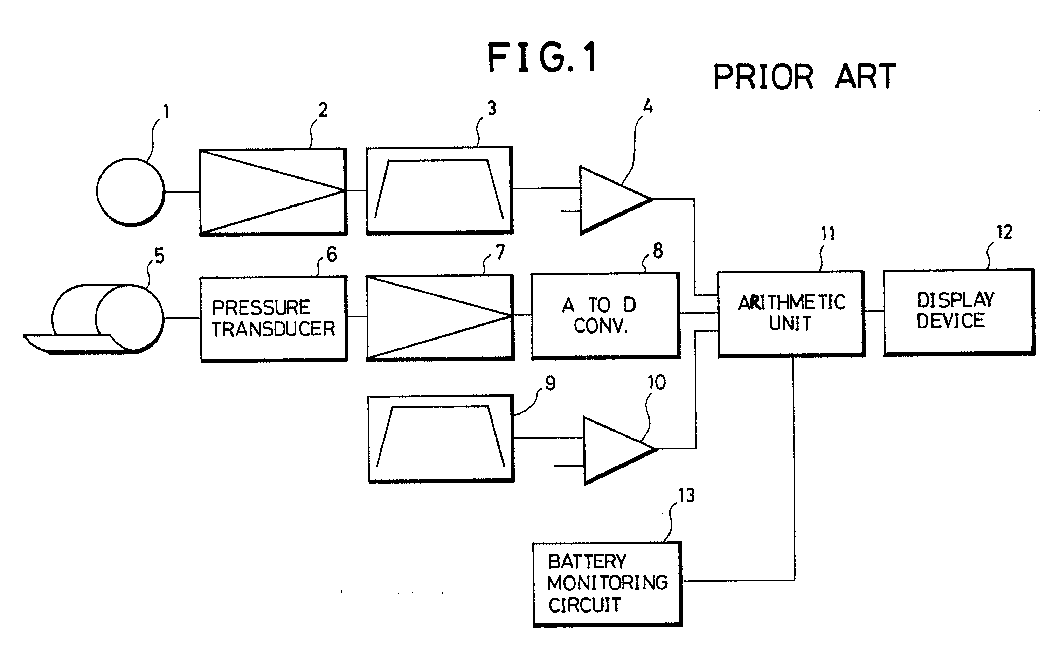

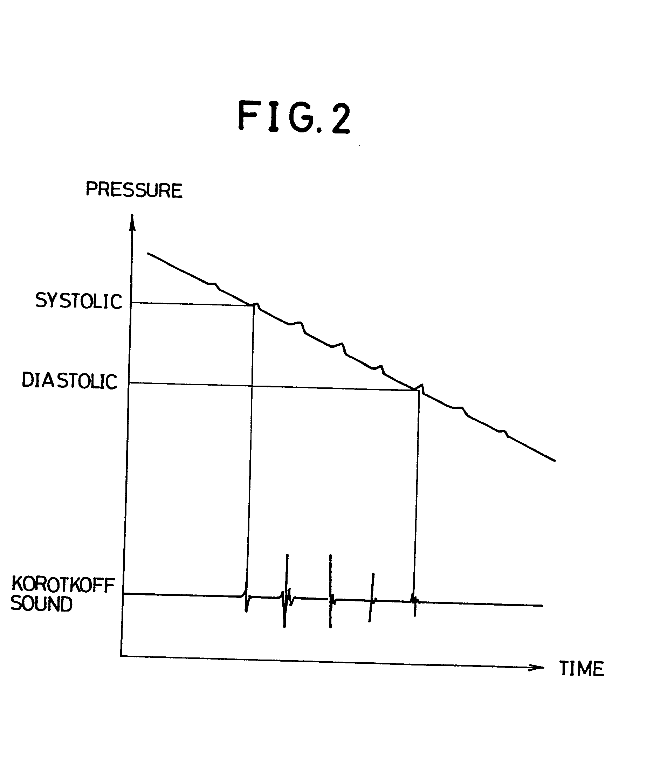

Figure 1 is a block diagram of a conventional electronic sphygmonanometer and Figure 2 is a graph illustrating the principle of measurement of blood pressure.

A cuff 5 wrapped about the upper arm is inflated to a pressure of more than the systolic pressure and then the pressure is allowed to decrease gradually. As the pressure decreases Korotkoff sound (called K-sound hereinafter) is produced in the artery. With further decrease in pressure, the K-sound disappears. The value of the pressure at the time when the K-sound is produced is defined as the systolic pressure and the value of the pressure at the time when the K-sound disappears is defined as the diastolic pressure. The K-sound is detected by a microphone 1 and is discriminated from noise by an amplifier 2 and a filter 3. The output of the filter 3 is converted to a pulse signal by a comparator 4.

The pressure in the cuff 5 is converted to a voltage by a pressure transducer 6 whose output voltage is amplified by an amplifier 7. The amplified voltage is read into an arithmetic unit 11 through an analog-to-digital convertor 8.

The pressure in the cuff 5 varies somewhat due to the pulse pressure during the production of the K-sound. Such variation is distinguished by a filter 9 and a comparator 10 produces a reference pulse signal at its output terminal. The determination of the existence of K-sound is performed by the reference pulse signals so that the measurement accuracy of the blood pressure is improved. The arithmetic unit 11 determines systolic and diastolic pressures which are then displayed by a display device 12. A battery monitoring circuit 13 detects fall in battery voltage.

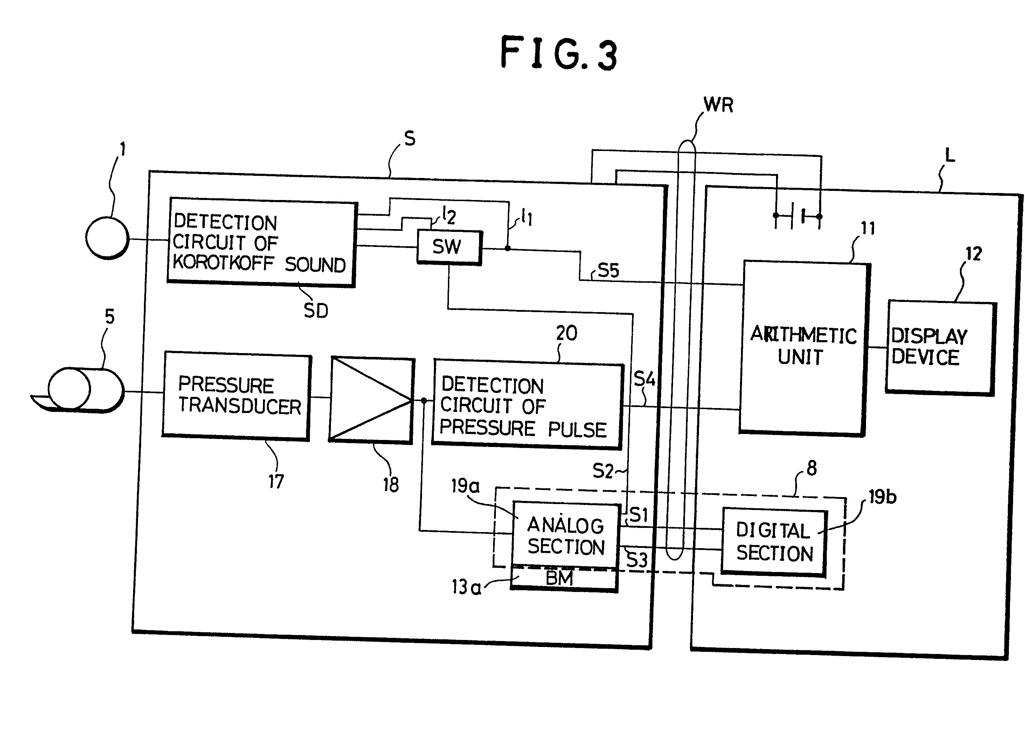

Figure 3 is a block diagram of one embodiment of an electronic sphygmonanometer according to the present invention. The sphygmonanometer consists of an analog detection circuit S, a digital logic circuit L, a signal line section WR connected between the detection circuit S and the logic circuit L, and the microphone 1 of the cuff 5 connected to the detection circuit S.

The detection circuit S includes a Korotkoff (K-) sound detection circuit SD, a semiconductor pressure transducer 17, an amplifier 18, a pressure pulse detection circuit 20 and an analog section 19a of the analog-to-digital convertor 8.

The logic circuit L includes the arithmetic unit 11, a digital section 19b of the analog-to-digital convertor 8 and the display device 12. The detection circuit S is formed on a bipolar IC chip and the logic circuit L is formed on a C-MOS IC chip. The signal line section WR includes a signal line S5 connected through a switch circuit SW so that the signal line S5 is a bidirectional line between the detection circuit SD and the arithmetic unit 11. A signal line S4 is connected between the pressure pulse detection circuit 20 and the arithmetic unit 11. Signal lines S1,S3 are connected between the analog section 19a and a digital section 19b. The detection circuit S is used as a transmitter, and the logic circuit L is used as a receiver.

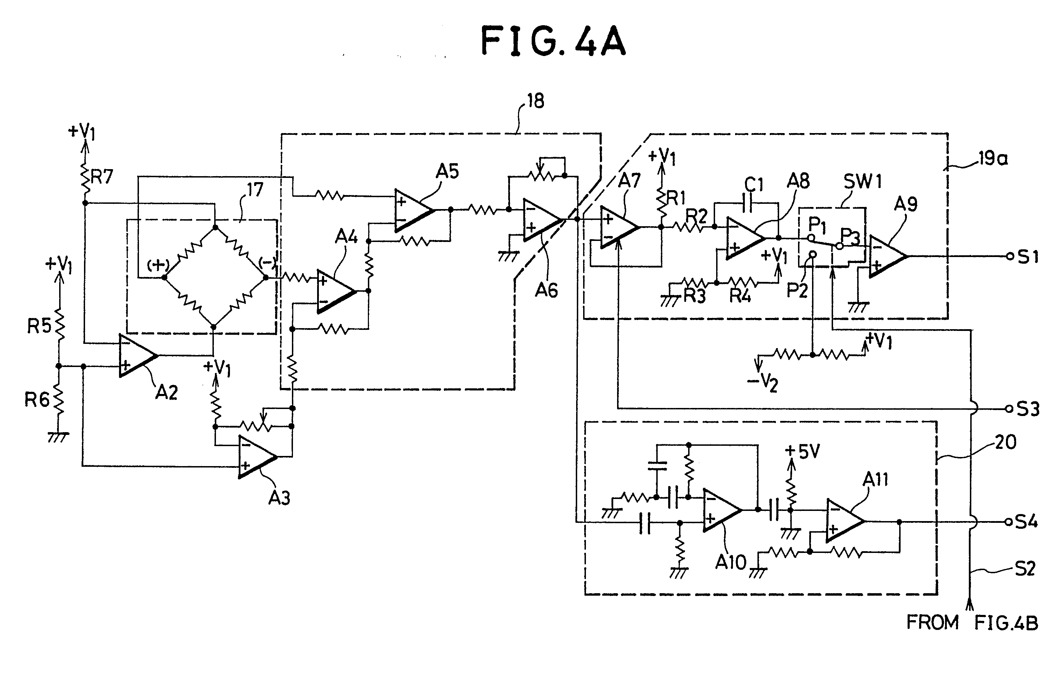

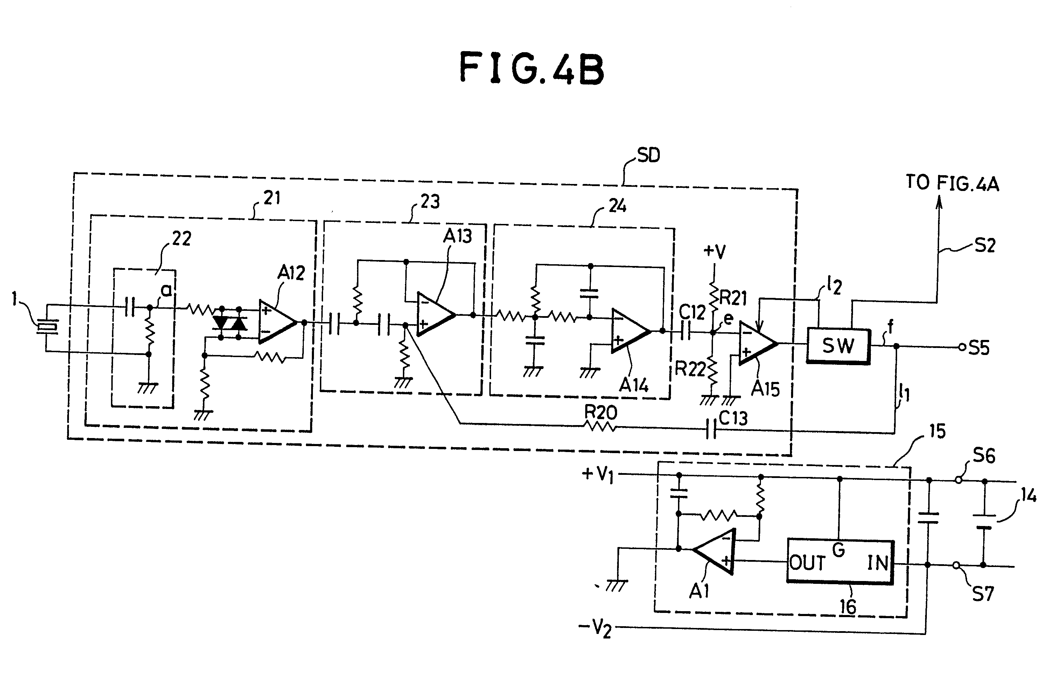

Referring now to Figures 4A and 4B, Figure 4A is a circuit diagram of the detection circuit S and Figure 4B is a circuit diagram of the K-sound detection circuit SD and a reference voltage circuit 15. The detection circuit S is constructed of bipolar transistors, which are actuated by about 3 volts. In Figure 4B the reference voltage circuit 15 produces a voltage of 1.2 volts at its output terminals. The positive terminal of a battery 14 is of 1.2 volts (referred to as +V1 hereinafter) and the negative terminal is of unstable voltage (referred to as -V2 hereinafter).

The reference voltage 15 is comprised of a reference voltage circuit 16 and an operational amplifier A1. The reference voltage circuit 16 produces a voltage using the energy gap of a semiconductor and the voltage is lowered by 0.6 volts as compared with the voltage +V1. The reference voltage is produced by the amplifier A1.

The transducer 17 (Figure 4A) is driven with a constant current by an operational amplifier A2. The value of the constant current is +V1.R5/(R5+R6).R7 determined by the voltage +V1 and the resistance of the resistors R5, R6, R7, the driving current being proportional to the sensitivity of the pressure and the resistance of the pressure transducer being related to the sensitivity of the pressure.

The amplifier 18 includes low offset type operational amplifiers A4 to A6 and an amplifier A3 is used for adjusting the offset voltage. The signal output of the amplifier 18 is applied to the analog section 19a of the analog-to-digital converter 8 and the pressure pulse detection circuit 20. The analog section 19a of the analog-to-digital convertor 8 is a double integral type integrator, and is charged while the signal line S3 is at high level and discharged at constant current while the signal line S3 is at low level.

The analog section 19a comprises an operational amplifier A7 which functions as a voltage follower when the signal line S3 is at high level. The signal voltage and the amplifier 18 has the same polarity as the voltage -V2 and the charge current of the amplifier 18 is determined by a resistor R2. A resistor R1 determines the bias current of the output stage of the operational amplifier A7. The operational amplifier A7 is completely cut off when the signal line S3 becomes low level, and the discharge current which is of opposite polarity to the polarity of the voltage +V1 flows through the resistors R1, R2 to a capacitor C1. An operational amplifier A8 which functions as an integrator receives a voltage E1 divided by resistors R3, R4 at its non-inverting input terminal. A comparator A9 is used for not only determining the output of the operational amplifier A8 but also for monitoring the battery voltage of the battery 14.

Referring to the detection circuit 20, the signal voltage of the operational amplifier A6 is applied to the pressure pulse detection circuit 20 serving as a filter to produce a reference pulse in synchronism with pulse pressure. An operational amplifier A10 consists of a quadratic (second order) band pass filter and a low pass filter.

Referring to the K-sound detection circuit SD, the microphone 1 is of the piezoelectric type which produces relatively large output. The output of the microphone 1 is amplified to a predetermined voltage by an amplifier 21. The amplifier 21 includes a high pass filter 22 to prevent an operational amplifier A12 from saturating, and functions as an impedence match. The noise component is eliminated by a quadratic high pass filter 23 and a quadratic low pass filter 24. The quadratic low pass filter 24 is of the multi-feedback type and has an amplifying operation and the output thereof is fed to a comparator A15 which produces digital pulses at its output terminal.

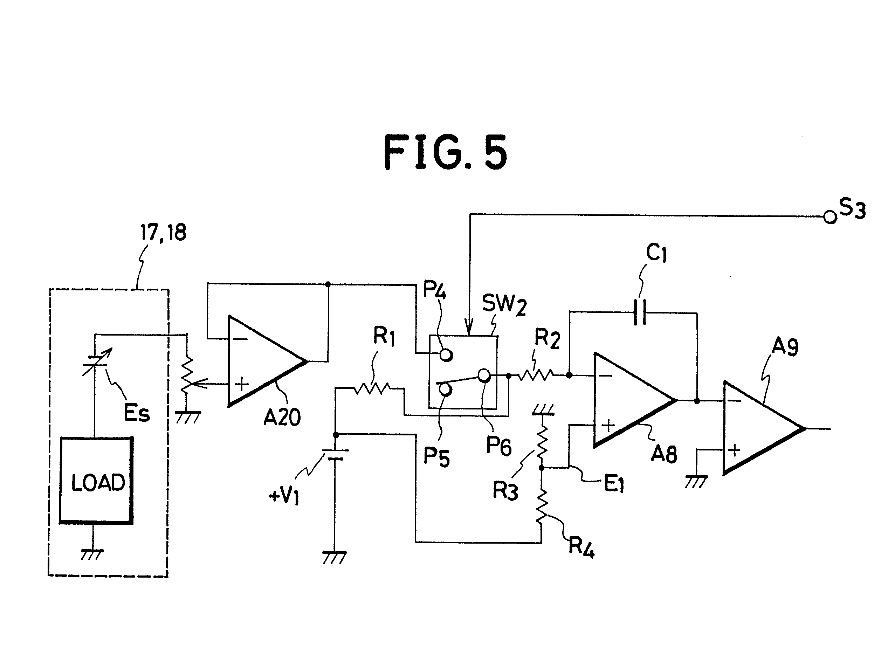

Figure 5 is a circuit diagram to explain the operational amplifier A7 of Figure 4A. The amplifier A7 has the same function as an operational amplifier A20 and a switch SW2, that is to say, it operates as an electronic switch to change the charge current of the double integral type analog-to-digital convertor to the discharge current and vice versa.

A terminal P6 of the switch SW2 is connected to a terminal P4 when the signal line S3 is high level and the operational amplifier A20 functions as a voltage follower. The capacitor C1 is charged by the charge current which is determined by the input voltage of the resistor R2 at the charge time, whereas the capacitor C1 is discharged by the discharge current which is determined by the resistors R1,R2 at the discharge time. At the discharge time, the switch SW2 is controlled to connect the terminal P6 to the terminal P5 by making the signal line S3 low level.

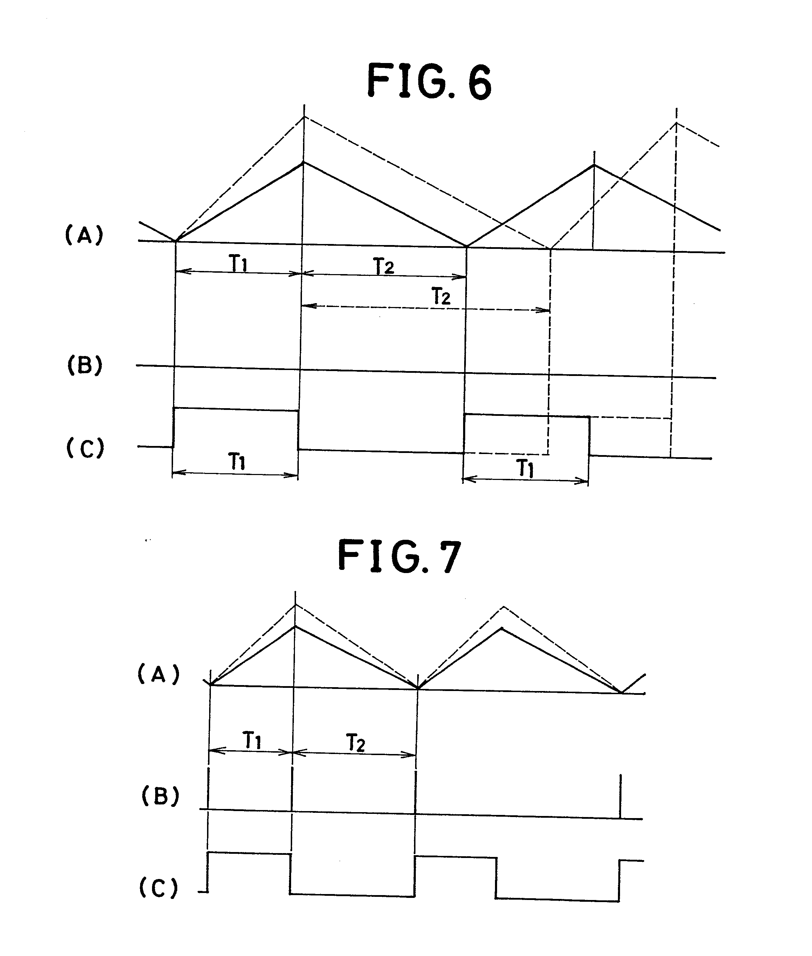

Figure 6 is a timing chart showing outputs of the double integral type analog-to-digital converter 8. In Figure 6, waveform (A) is the output of the operational amplifier A8, waveform (B) is the output of the comparator A9 and waveform (C) is the signal waveform of the signal line S3. T1 is the charge time and T2 is the discharge time.

The following equation is obtained because the quantity of electric charge to flow into the capacitor C1 in the charge time T1 is equal to the electric charge to flow out from the capacitor C1 in the discharge time T2.

$$\frac{Es}{R2} \cdot T1 = Id \cdot T2 = \frac{+V1}{R1+R2} \cdot T2 \quad \ldots\ldots\ldots (1)$$

where Es is a signal voltage which is the output voltage of the operational amplifier A6 and $Id = Er/R1+R2$ is the reference discharge current. Assuming that the charge time T1 is constant, the signal voltage Es is found by measuring the discharge time T2.

On the other hand, the signal voltage Es is found from the following equation because the pressure sensitivity of the pressure transducer 17 is proportional to the constant current value:

$$Es = K \cdot \alpha \cdot Is \cdot P \quad \ldots \ldots (2)$$

where K is the total gain of the operational amplifiers A4 to A6, $\alpha$ is a coefficient of pressure sensitivity, P is the pressure and $Is = \dfrac{R5}{R5+R6} \cdot \dfrac{+V1}{R7}$ is the driving current of the pressure transducer 17.

Accordingly, from equations (1) and (2):

$$T2 = K \cdot \alpha \cdot \dfrac{R1+R2}{R2} \cdot \dfrac{R5}{(R5+R6) \cdot R7} \cdot P \cdot T1 \quad \ldots \ldots (3)$$

The discharge time T2 does not depend on the voltage +V1 of the reference voltage circuit 15, that is to say, the variation of the reference voltage does not affect the accuracy of pulse pressure measurement. This is explained with reference to Figure 7, the solid line showing the slope with non-variation of the reference voltage and the dotted line showing the slope with variation of the reference voltage.

The pressure transducer 17 increases its current value and its pressure sensitivity is increased when the variation of the reference voltage is increased. The charge current increases as indicated by the dotted line but the reference discharge current increases simultaneously. As a result, the discharge time T2 does not vary. The discharge time T2 does not vary, even if $E1 = \dfrac{R3}{R3+R4} \cdot (+V1)$ is applied to the non-inverting input terminal of the operational amplifier A8 as shown in Figure 5.

In Figure 7, waveform (A) is the output of the operational amplifier A8, waveform (B) is the output of the comparator A9 and waveform (C) is the signal waveform of the signal line S3.

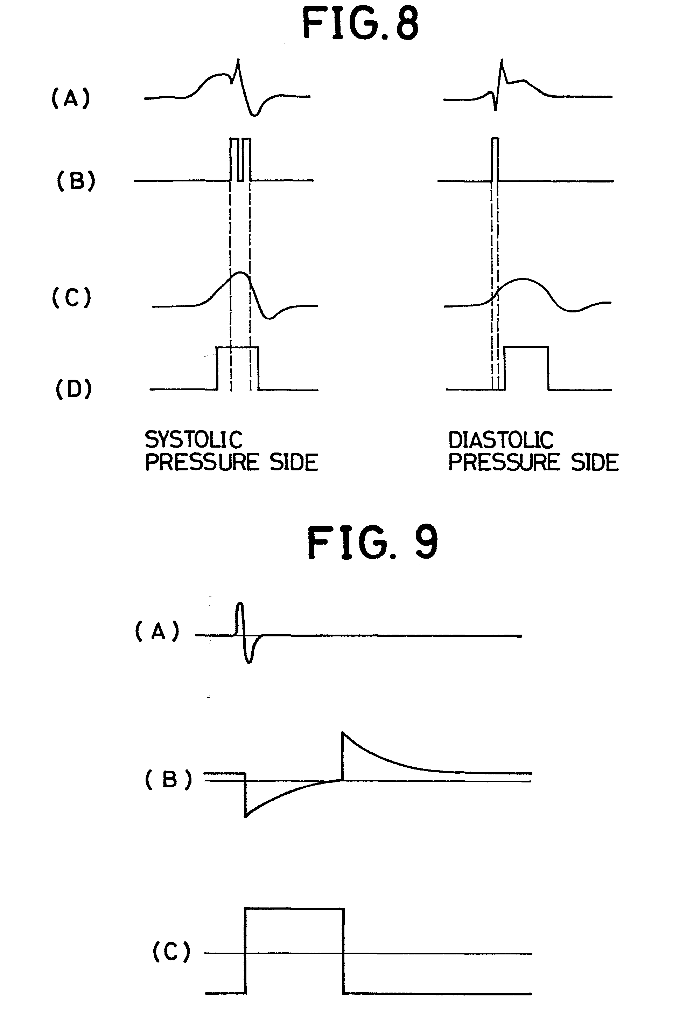

Figure 8 shows the K-sound and the pressure pulse waves having removed a feedback circuit consisting of a resistor R20 and a capacitor C13 as shown in Figure 4B from the K-sound detection circuit SD.

Waveform (A) is the K-sound and waveform (B) is a K-sound pulse from the comparator A15 serving as a level detector. Waveform (C) is the output of a pressure pulse filter including the operational amplifier A10 and waveform (D) is a pressure pulse which is an output of a comparator A11 serving as a level detector.

K-sound is more in advance of the pressure pulse on the diastolic pressure side than on the systolic pressure side. On the diastolic pressure side, the K-sound pulse is not coincident with the pressure pulse as shown in Figure 8.

In Figure 4B, the K-sound detection circuit SD includes the feedback circuit consisting of the resistor R20 and the capacitor C13 for enlarging the pulse width of the K-sound pulses. In Figure 4b and Figure 9, the quadratic low pass filter 24 produces a negative pulse $e$ as shown by waveform (B) when the pressure pulse is applied to the microphone 1. The output as shown by waveform (C) of the comparator A15 goes positive when the output of the quadratic low pass filter 24 is over the detection level determined by resistors R21, R22. The output of the comparator A15 is equal to an output $f$ of the switch circuit SW. This output $f$ is in positive feedback through the capacitor C13 and the resistor R20 to the quadratic high pass filter 23.

Accordingly, the output of the quadratic low pass filter 24 becomes negative because the direct current bias of the non-inverting input terminal of an operational amplifier A13 of the quadratic high pass filter 13 is changed when the output $f$ of the comparator A15 becomes positive. The voltage level of the non-inverting terminal of the comparator A15 goes upward with a time constant determined by the resistors R21, R22 and a capacitor C12. The output of the comparator A15 recovers its initial state so that it produces a one-shot pulse at least. The width of the one-shot pulse depends on the value of the resistors R21, R22, and the capacitor C12 but does not depend on the feedback resistor R22 and the capacitor C13.

In this manner, the K-sound detection circuit SD produces the appropriate pulse as shown by waveform C in Figure 9.

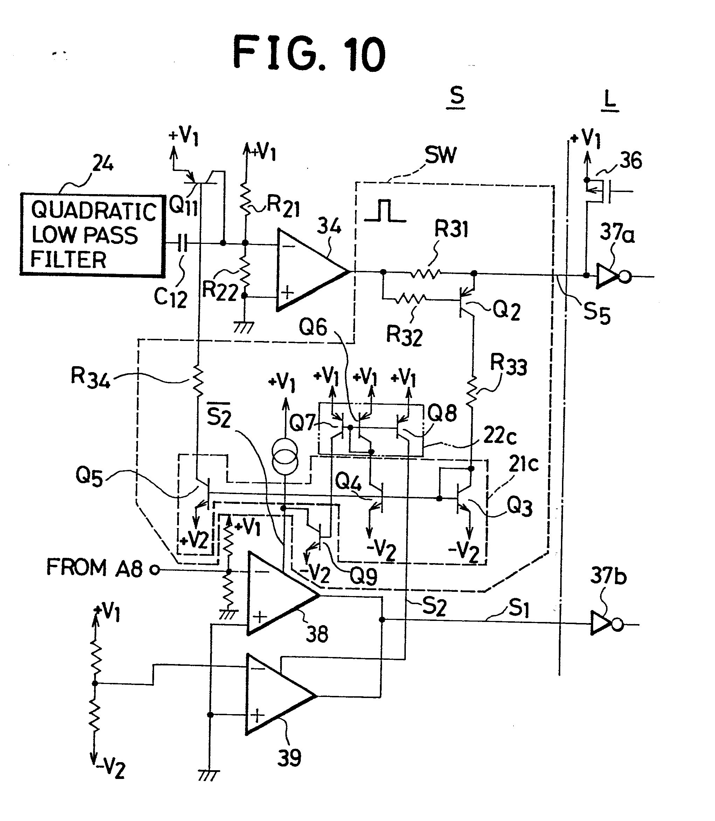

Figure 10 is a circuit diagram for decreasing the number of signal lines connected between the detection circuit S and the logic circuit L. The signal line S5 is used as a bidirectional line. In blood pressure measurement, a pulse signal is produced on the signal line S5 from a comparator 34 and synchronises with the K-sound. However, the result of battery voltage determination from the battery monitoring circuit 13 appears on the signal line S1 by pulling up the voltage of the signal line S1 to the power source voltage of the logic circuit L serving as a receiver. In Figure 10 the K-sound of the quadratic low pass filter 24 is outputted as a digital pulse from the comparator 34. The output of the comparator 34 is transmitted through a resistor R31 to a C-MOS element 37a. There is no voltage drop across the resistor R31 because current does not flow in the resistor R31 when a C-MOS element 37a has a high resistance. For this reason, a transistor Q2 is off.

Transistors Q3, Q4, Q5 constitute a current mirror circuit 21C and transistors Q6, Q7, Q8 constitute a current mirror circuit 22C. These current mirror circuits 21C, 22C reflect (absorb and flow out) current of the same amplitude as the collector current of the transistor respectively. However, the current mirror circuits 21C, 22C do not operate while the transistor Q2 is off. Accordingly, transistors Q11 and Q9 are off. Comparators 38, 39 are cut off when a bias current is not provided to them.

At the time of blood pressure measurement, the comparator 38 is operative and the comparator 39 is inoperative. The comparator 38 receives the pulse pressure signal through the operational amplifier A8 and the output of the comparator 38 is transmitted to a C-MOS element 37b.

Where the signal line S5 is pulled up, the output of the comparator 34 is low level in the normal state. In the normal state, the current flows from the digital section 19b of the logic circuit L to the analog section 19a of the detection circuit S, on the signal line S5. The transistor Q2 becomes ON in response to the voltage across the resistor R31 and at the same time the current mirror circuits 21C, 22C operate.

When the current mirror circuits 21C, 22C operate, the transistor Q9 becomes ON and a bias current is not provided to the comparator 38 but the current from the current mirror circuit 22C

is provided to the comparator 39 which serves as part of the battery monitoring circuit, the determination of which appears on the signal line S1.

When the output of the comparator 34 becomes high level, the voltage level at one terminal of the resistor R31 is equal to that at the other terminal so that the transistor Q2 becomes OFF. To avoid this the output of the comparator 34 is maintained low level by making the transistor Q11 ON.

### CLAIMS

1. An electronic sphygmonanometer comprising: a K-sound detection circuit (SD) for detecting Korotkoff sound from a microphone (1); a pressure transducer (17) for converting pressure to an electric signal; a pressure pulse detection circuit (20) for detecting a pressure pulse signal from said electric signal, the detection circuit being connected to an amplifier (18) through said pressure transducer; a double integral type convertor (8) for converting an analog output of the amplifier (18) to a digital signal; an arithmetic unit (11) for receiving the output signals of said K-sound detection circuit (SD), said pressure pulse detection circuit (20) and said convertor (8); and a display device (12) for indicating systolic pressure and diastolic pressure digitally, characterised by detection means (S) including said K-sound detection circuit (SD), said pressure transducer (17), said amplifier (18), said pressure pulse detection circuit (20) and an analog section (19a) of said convertor (8), a logic means (L) including a digital section (19b) of said convertor (8), said arithmetic unit (11) and said digital display (12), and a plurality of signal lines (WR) connected between said detection means (S) and said logic means (L) and including a first signal line (S5) for transmitting an output signal of said K-sound detection circuit (SD) to said arithmetic unit (11), a second signal line (S4) for transmitting said output of said pressure pulse detection circuit (20) to said airthmetic section (19a) and a third signal line (S1,S3) for transmitting an output of said analog section (19a) to said digital section (19b).

2. An electronic sphygmonanometer as claimed in claim 1 characterised in that said detection means (S) and said logic means (L) are formed on different IC chips.

3. An electronic sphygmonanometer as claimed in claim 1 or 2 characterised in that said signal line (S5) is a bidirectional signal line.

4. An electronic sphygmonanometer as claimed in any preceding claim characterised in that said analog section (19a) includes an electronic switch (SW2) for switching between charging current and discharging current, said electronic switch comprising a voltage follower having a transistor, and an integrator (A8) having an integral capacitor, a first resistor (R1) and a second resistor (R2) connected in series, said integrator (A8) having one input terminal connected to the series arrangement of said first and second resistors (R1,R2) and the other input terminal connected to a reference voltage source (E1,R3,R4).

5. An electronic sphygmonanometer as claimed in any preceding claim characterised in that said K-sound detection circuit (SD) includes filter means (23) for eliminating noise, a level detection circuit (A15) for determining level of detection of Korotkoff sound and connected to said filter and a feed back means (R20,C13) connected between an output of said level detection circuit and said filter means (23).

6. An electronic sphygmonanometer as claimed in any preceding claim characterised in that, in operation, discharge current value used for said convertor (8) is proportional to the value of the constant current used for said pressure transducer.

7. In an electronic sphygmonanometer having detection circuit means (SD) for detecting Korotkoff sounds connected to a microphone (1), a pressure transducer (17) for converting a pressure to an electric signal, a detection circuit (20) for detecting a pressure pulse signal from said electric signal connected through an amplifier (18) to said pressure transducer, a double integral type integrator (8) for converting said electric signal of analog value to an digital signal of digital value, an arithmetic unit (11) for receiving the output signals of said detection circuit means, said detection circuit and said double integral type integrator, and a display device for indicating systolic pressure and diastolic pressure digitally, the improvement comprising an detection section including said detection circuit means, said pressure transducer, said amplifier, said detection circuit and an analog section (19a) of said double integral type integrator, a logic section including a digital section (19b) of said double integral type analog-to-digital convertor, said arithmetic unit and said digital display, and a plurality of signal lines (WR) connected between said detection section and said logic section and including a first signal line (S5) for transmitting said output signal of said detection means to said arithmetic unit, a second signal line (S4) for transmitting said output of said detection circuit to said arithmetic unit and third signal line (S1,S3) for transmitting an output of said analog section to said digital section of said double integral type analog-to-digital convertor.

FIG.1  PRIOR ART

# FIG.2

0145208

# FIG.3

# FIG.4A

FROM FIG.4B

FIG.4B

# FIG. 5

0145208

# FIG. 6

(A)

T1    T2

T2

(B)

(C)    T1                    T1

# FIG. 7

(A)

T1    T2

(B)

(C)

0145208

# FIG.8

(A)

(B)

(C)

(D)

SYSTOLIC
PRESSURE SIDE

DIASTOLIC
PRESSURE SIDE

# FIG. 9

(A)

(B)

(C)

# FIG. 10

**European Patent Office**

**EUROPEAN SEARCH REPORT**

EP 84 30 7381

## DOCUMENTS CONSIDERED TO BE RELEVANT

| Category | Citation of document with indication. where appropriate. of relevant passages | Relevant to claim | CLASSIFICATION OF THE APPLICATION (Int. Cl.4) |
|---|---|---|---|
| X | US-A-4 290 434 (W. JEWETT)<br><br>* Column 8, line 42 - column 14, line 60; figures 7 - 11e * | 1,8 | A 61 B 5/02<br>A 61 B 7/04 |
| A | | 2 | |
| A | IEEE TRANSACTIONS ON CONSUMER ELECTRONICS, vol.CE-27, no.1, February 1981, pages 91-101, NEW YORK, (US). D. BRAY: "Bipolar semi-custom ICs in non entertainment consumer applications".<br><br>* The whole document * | 1,2,5, 8 | |
| A | HERMANN SCHMID:"ELECTRONIC ANALOG-DIGITAL CONVERSIONS 1970, Van Nostrand Reinhold Co. NEW YORK, (US).<br><br>* Pages 282-295 * | 1,4,7, 8 | **TECHNICAL FIELDS SEARCHED (Int. Cl.4)**<br><br>A 61 B |
| A | US-A-3 978 848 (D. YEN et al.)<br><br>* Column 2, line 19 - column 3, line 37; figure 1 * | 1,8 | |

The present search report has been drawn up for all claims

| Place of search | Date of completion of the search | Examiner |
|---|---|---|
| THE HAGUE | 15-02-1985 | ZILLIOX J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82